# EUROPEAN PATENT APPLICATION

(11) **EP 3 100 701 A1**
(43) Date of publication of application: **07.12.2016**
(21) Application number: 15170736.1
(22) Date of filing: 04.06.2015
(51) Int. Cl.: A61F 2/24

(54) **MITRAL VALVE STENT WITH ANTERIOR NATIVE LEAFLET GRASPING AND HOLDING MECHANISM**

(71) Applicant: Epygon Sasu, 75008 Paris (FR)
(72) Inventor: PASQUINO, Enrico, 10020 Marentino (IT); SCORSIN, Marcio, 10125 Torino (IT); GARD, Marco, 10031 Borgomasino (Torino) (IT); CASALEGNO, Sergio, 10099 San Mauro Torinese (Torino) (IT)
(74) Representative: Grosfillier, Philippe

(57) **Abstract**

Mitral valve stent (1) having an asymmetrical tubular shape, with a sub annular anterior side (2) of shorter height than the height of the sub annular posterior side (3), the sub annular anterior side (2) comprising a self-folding native leaflet engagement member (4-9) which forms an extension of said sub annular anterior side (2) when the stent (1) is collapsed and which is oriented in another direction when the stent (1) is in an expanded state.

## Description

### Field of invention

The present invention refers to mitral valve stents and more precisely to such stents that comprise a native leaflet locking mechanism.

### State of the art

A common surgical procedure to position a mitral heart valve prosthesis is the transcatheter one, i.e. the prosthesis is a stent that is introduced in a collapsed state through a catheter. The stent expands when it leaves the catheter, just before reaching its definitive position.

Usually, when a standard replacement is performed, the native mitral valve is resected or partially resected to allow the heart prosthesis work properly and not be disturbed by the subvalvular apparatus. When a transcatheter mitral valve prosthesis is used to replace the native valve, resection is not feasible and the native valve must stay in place. Keeping the native valve may offers several advantages, mainly in terms of ventricular function because the mitral valve is attached to the ventricular wall and actively participate in the ventricular contraction. However, the presence of the native anterior leaflet (which has no use anymore), may obstruct the left ventricle outflow tract (LVOT). Indeed, by implanting a stent in the mitral position, the metallic structure forming the stent creates a radial force to secure the valve in place. Consequently pushing the anterior leaflet of the mitral valve towards the aortic valve may potentially lead to an obstruction of the LVOT. One strategy to overcome this problem is to positioning the stent very high, i.e. upstream, above the annular plane so as the ventricular part can be very short. The atrialization of the prosthesis reduces the risk of LVOT obstruction, but increases the risk of atrial thrombosis.

Another solution is to create engagement arms, as disclosed for instance in US patent application US 2011/208297 A1, to catch and block the valve native leaflets. However, even though an engagement arm could efficiently block the mitral leaflets, the more the stent protrudes deep down within the ventricle, the higher is the risk of LVOT obstruction. This risk is particularly elevated in some examples disclosed in US 2011/2008297 A1 where the height of the stent anterior side may is longer than the height of the posterior side.

Providing a stent anterior side of shorter height than the posterior side has been disclosed in international patent application WO 2013/160439 A1.
This prior art also discloses an anterior native leaflet locking mechanism. Initially the stent is symmetric and is made of a memory shape material. The anterior side is thermally everted and forms an engagement member for the anterior native leaflet.
This latest solution is however not entirely satisfactory. Everting the complete stent anterior side may negatively affect the stent properties in this region. The stent may be too rigid and/or too thick. This inconvenient also occurs at any symmetric or asymmetric segment of any stent, anterior, posterior or lateral.

There is therefore a need to improve the existing stent mitral valves.

### General description of the invention

The problems mentioned in the previous chapter may be overcome with a mitral valve stent according to the invention that has an asymmetrical tubular shape, with a sub annular anterior side of shorter height than the height of the sub annular posterior side, the sub annular anterior side comprising a self-folding native leaflet attachment member that forms an extension of said sub annular anterior side when the stent is collapsed and which is oriented in another direction when the stent is in an expanded state.

In the present document the expression "anterior side" or "stent anterior side" refers to the stent side that is directly facing the aortic valve when the stent is oriented in its definitive position within the native mitral valve complex. The "posterior side" refers to the stent side that is opposite to the anterior side.

The expression "sub annular" refers to a region that is below the annulus and within the ventricle.

The stent according to the invention is shorter at the sub annulus anterior side in order to reduce the risk of obstruction of the LVOT.

The stent is longer at the posterior side because it allows a better anchoring of the valve during systole.

In a preferred embodiment, in the collapsed state, the sub annular anterior side height added to the engagement member length is equivalent or shorter than the sub annular height of the posterior side. The presence of the engagement member does therefore not increase the stent length. This embodiment offers an important advantage. By not increasing the length of the stent and consequently the length of the delivery system necessary to release the valve, it facilitates a transcatheter approach through a transatrial access where the path to reach the mitral position is not straight.
When the stent is released, the engagement member bends, preferably of at least 160°. This creates an empty space at the anterior side and consequently do not obstruct the ventricular flow, and at the same time the anterior leaflet is grasped and taken away from the ventricular outflow tract.

In another embodiment the engagement member bends of 180°, or close to that value, when the stent is released.
The bending angle is predefined when the stent is manufactured.
Preferably the engagement member is made of a memory shape material. In this case the bending angle is thermally shaped.

In another embodiment, the engagement member is forming an integral part of the stent.

Advantageously there is only one single point that links the engagement member to the stent anterior side.

In another embodiment the engagement member is provided with a specific geometry containing at least one wavy line. Such a configuration allows a bending with a minimal torsion that avoids the damage of the metal crystalline structure and preserves the superelastic characteristics of the memory shape material.

In another embodiment the stent comprises a native leaflet locking system. Advantageously this locking system is defined by at least one extensions body, preferably two, bent out of preferably 30° from the stent structure. The locking system works in grabbing and retaining the native leaflet impeding its interference with the LVOT and providing thereby an efficient anterior anchoring to the valve. The locking mechanism is based on the sequence of events occurring during the release of the stent from the catheter. Initially the extension bodies are released and open, e.g. at 30°. Then the engagement member is released and bends out to a predefined angle, usually between 160-180°, in a way that moving upward the rim of the anterior mitral leaflet it allows the extension bodies to retain the native leaflet. Finally the native leaflet is pinched and retained between the extension bodies and the engagement members.

In another embodiment according to the invention the stent with comprises one anterior and one posterior engagement members which respectively block the anterior and the posterior mitral leaflets, both engagement members being located on the stent sub annular edge. The length of the anterior and posterior mitral leaflets is different. The anatomic distance between the anterior mitral annulus and the free edge of the anterior leaflet (middle of A2) is around 28mm. The distance between the posterior mitral annulus and the edge of the posterior leaflet (P2) is around 20mm. In case two engagement members are used to block both leaflets, they may be released at different level in the sub-annular stent structure. The anterior engagement member may be released further down because it needs to grab a longer leaflet. Otherwise, the posterior engagement member may be released at a higher level because it needs to grab a shorter leaflet.

### Detailed description of the invention

The invention is discussed below in a more detailed way with examples illustrated by the following figures:
Figure 1 shows an example of a mitral valve stent according to the invention.
Figure 2 is another view of the anterior side of the stent of figure 1.
Figure 3 shows a portion of a stent according to the invention, in a flat configuration.
Figure 4 represents native leaflet retained by a stent according to the invention.
Figure 5 represents the leaflet of figure 4 in a locked position.
Figure 6 shows different orientations of an engagement member according to the invention.
Figure 7 illustrates another example showing the fixation of an engagement member to the stent body.
Figure 8 shows a stent according to the invention in a collapsed state.
Figure 9 illustrates an example of the movement of an engagement member according to the invention, in an intermediate position.
Figure 10 shows the engagement member of figure 9 in a final position.
Figure 11 is a schematic representation of a stent according to the invention with one anterior and one posterior engagement members.
Figure 12 is a schematic representation of another stent according to the invention.
Figure 13 is a schematic representation of another stent according to the invention.
Figure 14 is a schematic representation of another stent according to the invention.
Figure 15 shows another engagement member according to the invention.
Figure 16 shows another engagement member according to the invention.
Figure 17 shows the engagement member of figure 16 with the extension bodies highlighted.
Figures 18 to 24 show other examples of engagement members according to the invention.

### Numerical references used in the figures :

1. Stent
2. Stent sub annular anterior side
3. Stent sub annular posterior side
4. Anterior native valve engagement member
5. Engagement member foldable portion
6. Wavy line
7. Bridge
8. Blade
9. Linking segment
10. Extension body
11. Posterior native valve engagement member
12.Anterior native leaflet

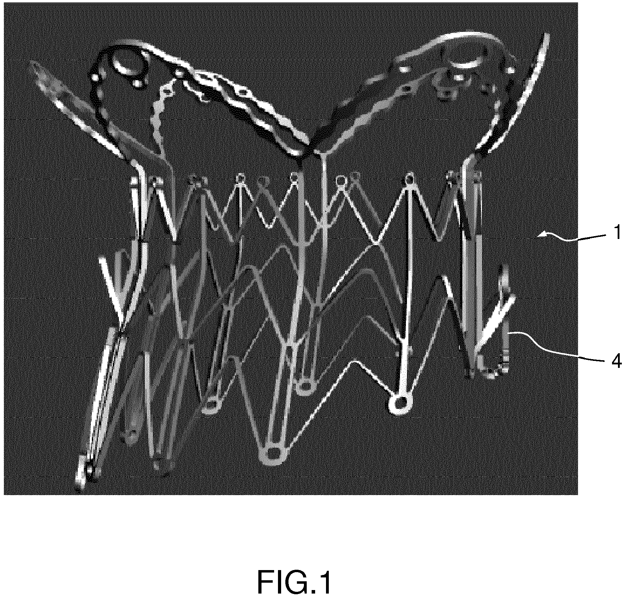

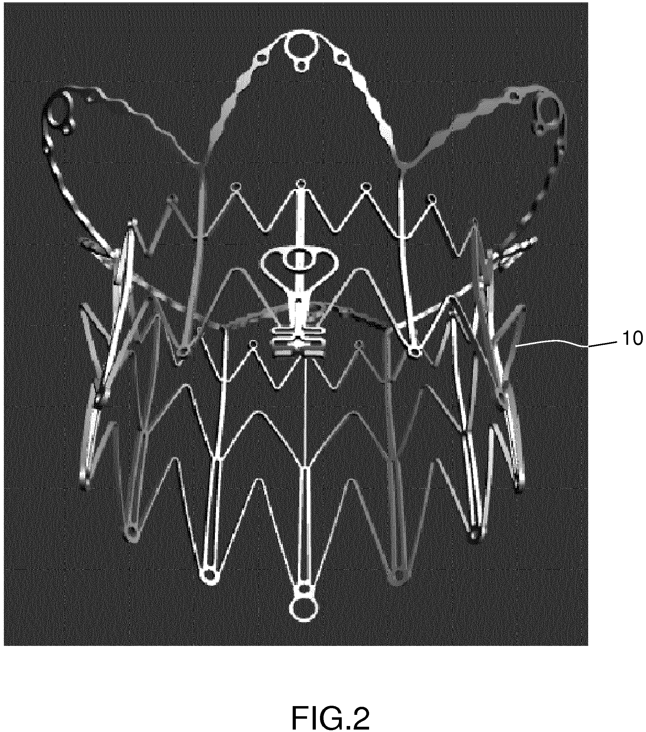

The sent **1** shown in Figure 1 is in an expanded state, with one engagement member **4** at the lateral side (commissure-commissure line) of the stent **1** (on the left side of the figure).
The engagement member 4 is oriented along a direction that is parallel to the wall of the stent body (see Figure 2).

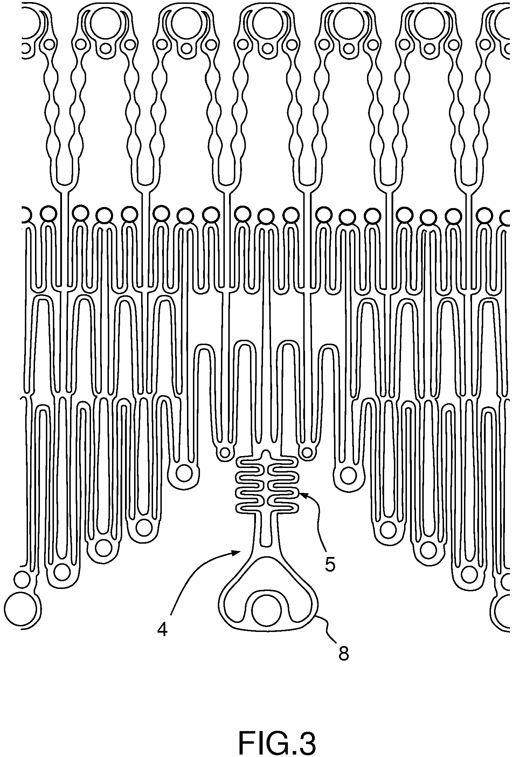

Figure 3 shows a portion of the stent **1,** in a collapsed state and flattened configuration, before its conformation into a collapsed state. The engagement member is in its flat status (0°angle) as it happens when the valve is loaded into the delivery system.

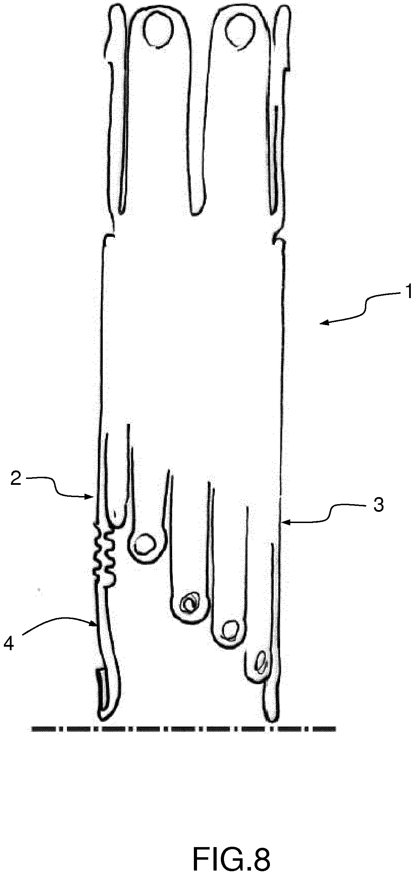

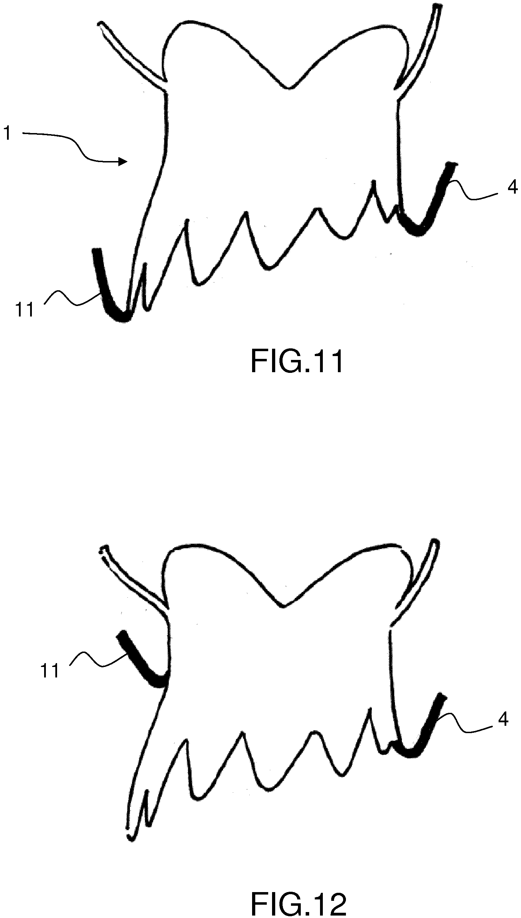

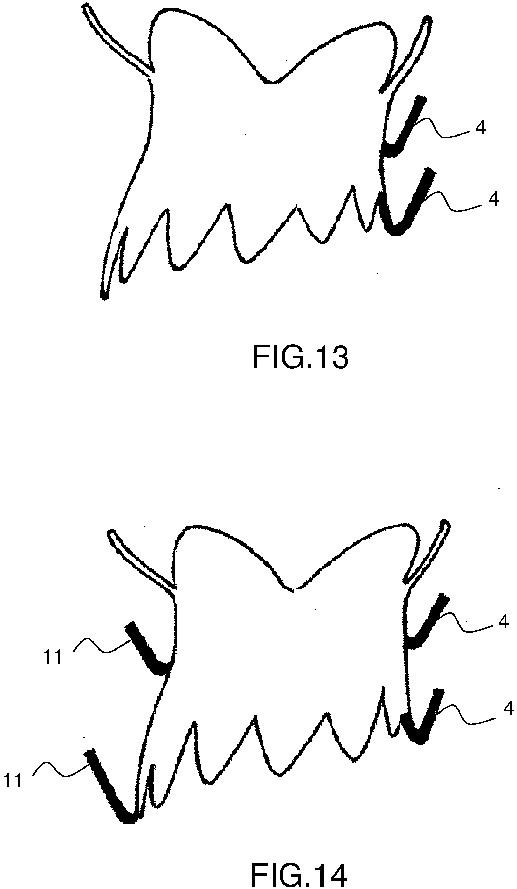

In this example the sub annular anterior side height added to the engagement member **4** length is equivalent or shorter than the sub annular posterior side height.

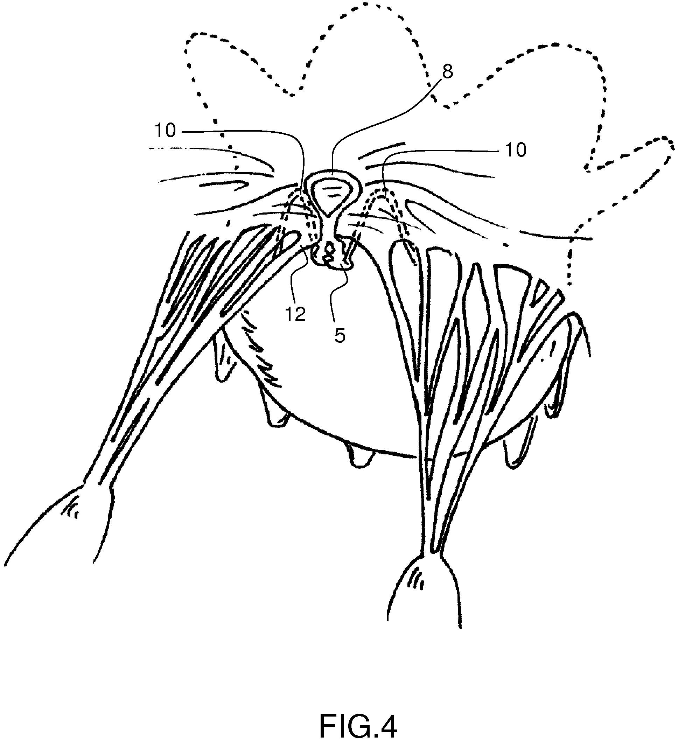

Figure 4 represents the grasping and retaining of an anterior native leaflet **12** at the middle leaflet segment A2, with an engagement member **4** and extension bodies **10** according to the invention. In this example the engagement member **4** locks the anterior leaflet **12** and the extension bodies **10** are placed behind it. This solution, retaining the middle segment A2 of the native leaflet **12,** is aimed at anchoring it in a portion where no chordae are present thus minimizing the risk of cordage ruptures.

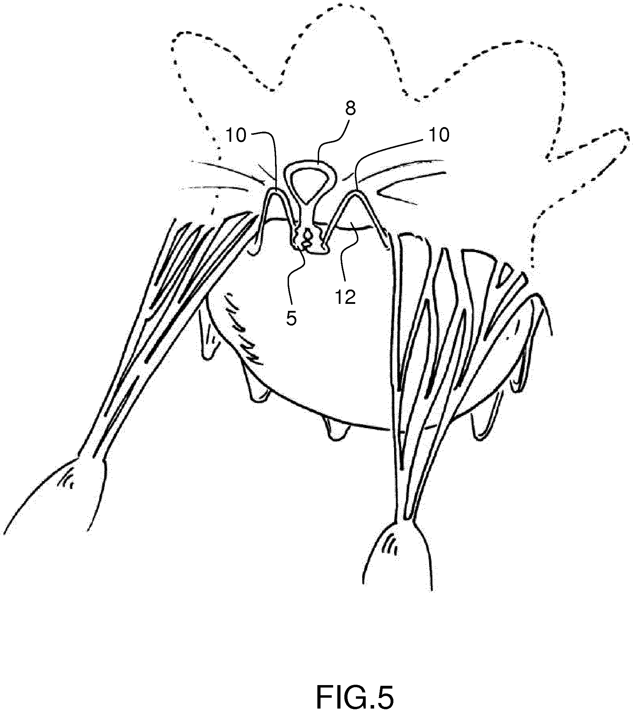

Figure 5 represents the leaflet **12** of figure 4 in a locked position. The locking mechanism is activated by the joint action of the extensions bodies **10** and the engagement member **4.** The extensions bodies grasp and hold the leaflet **12** and the engagement member **4** pinches and thus completely blocks the leaflet **12.** The engagement member **4** locks the anterior leaflet **12** and the extension bodies **10** are placed ahead of it. In both configurations the locking mechanism works.

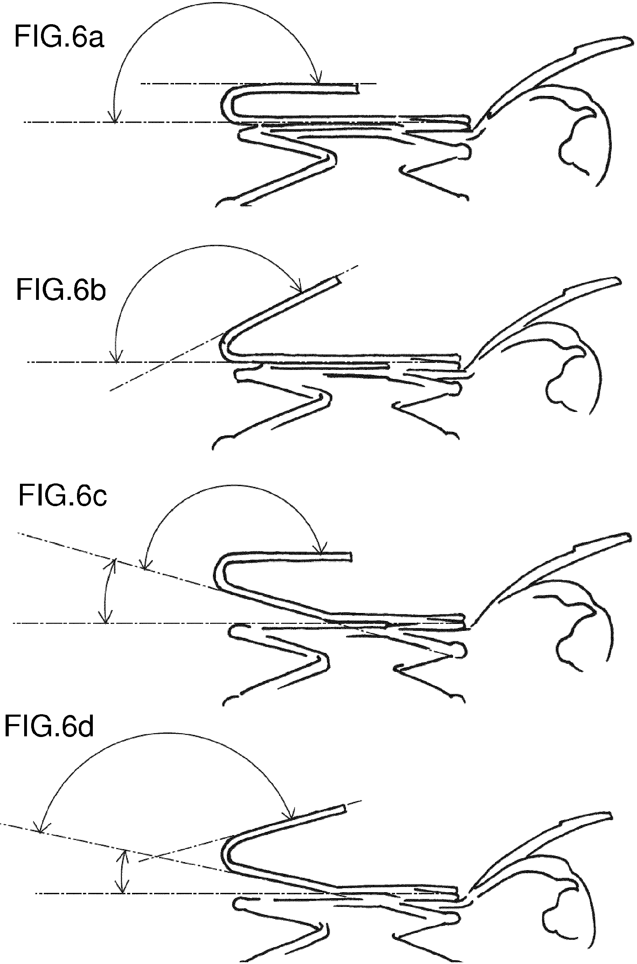

Figure 6 shows different orientations of the engagement member **4** when the stent **1** is in an expanded state. The engagement member **4** is part of the stent structure. In this figure different opening angles of the engagement member **4** are showed. In particular in picture 6c and 6d show that the stent pillar sustaining the engagement member **4** can be bent outward with a variable angle (0° to 40°) thus allowing it to be more effective in grabbing the anterior mitral leaflet **12.**

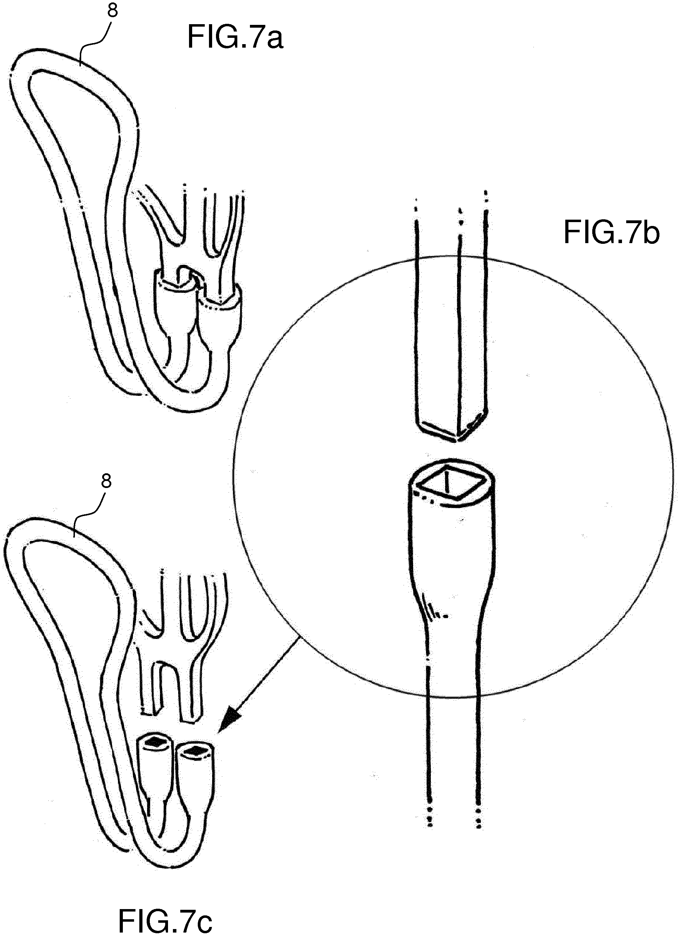

Figure 7 illustrates another example showing the fixation of an engagement member **4** to the stent body. In this case the engagement member **4** is not initially part of the stent structure. This is an alternative design solution in order to reduce the torsion of the engagement member **4** when is opened. This solution adopts the use of a wire, made of NiTinol or another metallic alloy, to be welded or crimped over supports obtained from the stent's structure. In this configuration the anchoring of the engagement member **4** to the stent **1** can be obtained with different technologies.
The sent **1** illustrated in Figure 8 is in a collapsed state. Here also the engagement member length does not increase the length of the stent **1.** The distal end of the engagement member **4** is at the same level of the distal end of the posterior segment of the stent **1.**

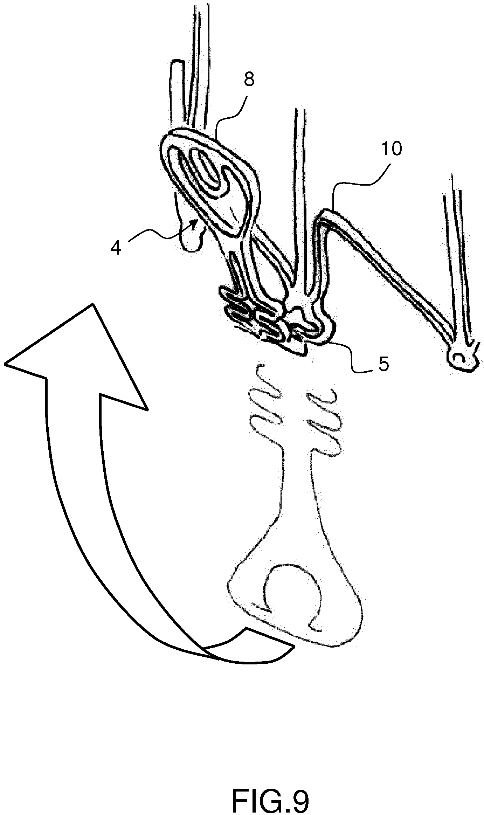

Figure 9 illustrates an example of the movement of the engagement member **4** when released from the delivery system according to the invention, in an intermediate position.

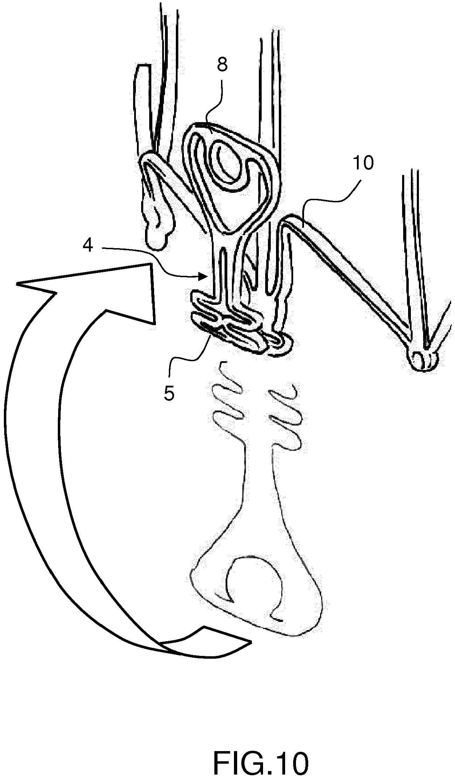

Figure 10 shows the engagement member **4** of figure 9 in a final position.
The stent **1** illustrated in Figure 11 contains one anterior and one posterior engagement members **4,11,** both being located on the distal end of the stent ventricular portion.
In the example of Figure 12 the stent **1** contains one anterior engagement member **4** located at the distal end of the anterior part of the stent ventricular portion and one posterior engagement member **11** located at mid height of the posterior side. This representation is in according with the anatomic characteristics of the mitral valve. The anterior leaflet **12** is longer than the posterior leaflet; consequently the engagement members **4,11** should be released at different heights.
In the example of Figure 13 two engagement members **4** are located on the stent anterior side.
In the example of Figure 14 the stent **1** contains two engagement members located **4** on the anterior side and two engagement members **11** located on the posterior side.

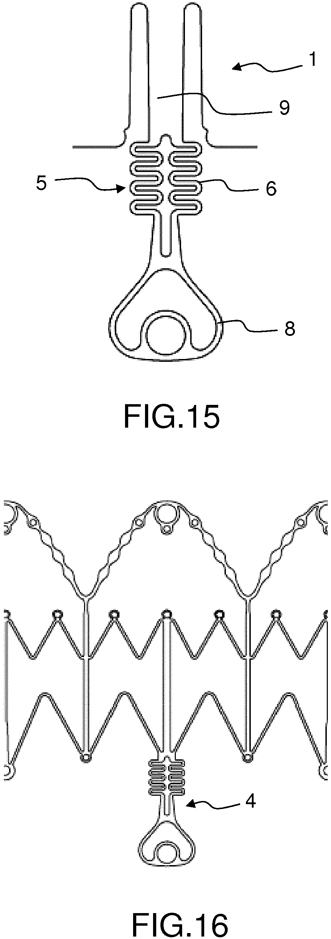

Figure 15 shows another engagement member **4** according to the invention. In this case the engagement member **4** is fixed to the stent **1** through a segment **9.**
Figure 16 shows another engagement member **4** according to the invention, which extends from the stent body. The engagement member **4** is part of the same memory shape material, e.g. nitinol, than the one which constitutes the stent body. Such a configuration prevents from any risk of corrosion or galvanic forces.

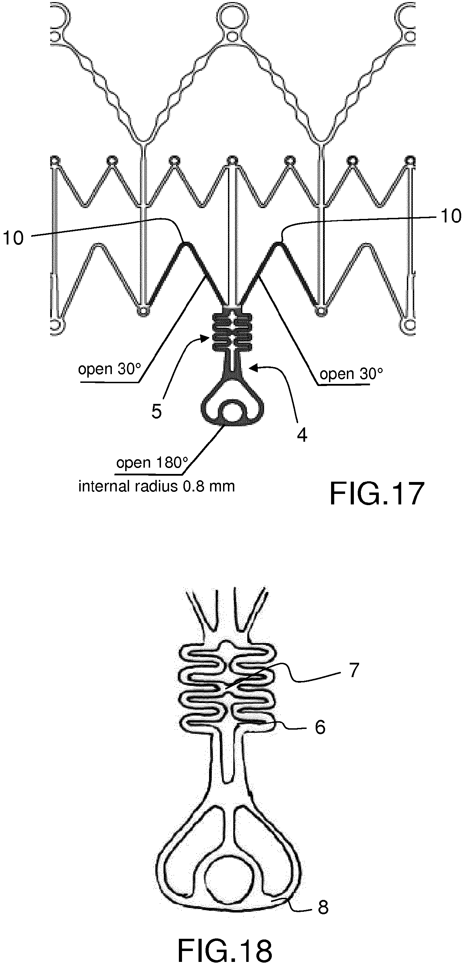

Figure 17 shows the engagement member **4** of figure 16 with the extension bodies **10** highlighted.
Figure 18 shows another engagement member according to the invention. In this example the wavy lines **6** are linked to each other through a bridge **7.**

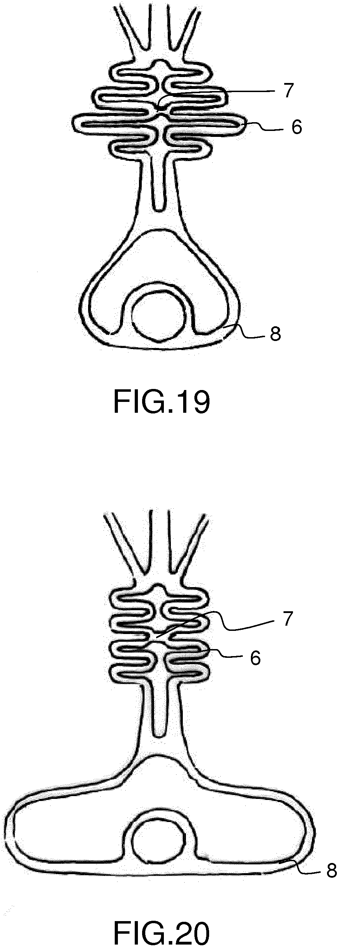

Figure 19 shows another engagement member according to the invention. The wavy lines **6** are linked to each other and asymmetric. The continuity with the anterior part of the stent is made by three elements, a main one and two secondary.
Figure 20 shows another engagement member according to the invention.

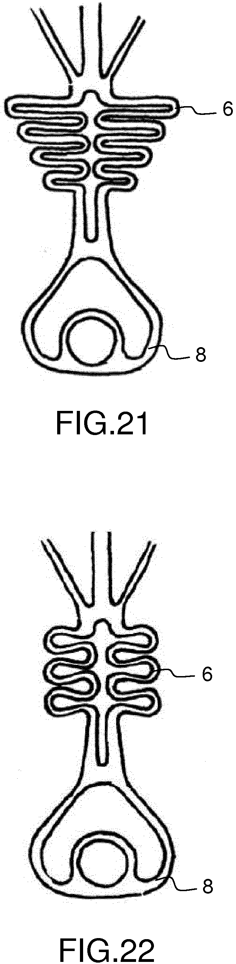

Figure 21 shows another engagement member according to the invention.
Figure 22 shows another engagement member according to the invention.

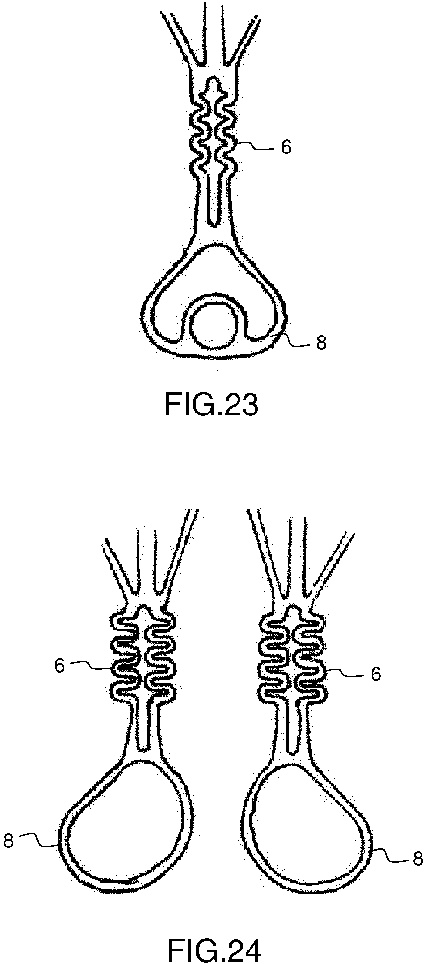

Figure 23 shows another engagement member according to the invention. The wavy lines **6** in this example are less pronounced and more linear.
Figure 24 shows two engagement members **4** according to the invention, the blades **8** are symmetrically oriented along different directions.

The invention is of course not limited to the illustrated examples. Any suitable geometry or material can be used for the stent and the engagement member(s).

## Claims

1. Mitral valve stent (1) having an asymmetrical tubular shape, with a sub annular anterior side (2) of shorter height than the height of the sub annular posterior side (3), the sub annular anterior side (2) comprising a self-folding native leaflet engagement member (4-9) which forms an extension of said sub annular anterior side (2) when the stent (1) is collapsed and which is oriented in another direction when the stent (1) is in an expanded state.

2. Mitral valve stent (1) according to claim 1 wherein said engagement member (4-9) is made of a memory shape material.

3. Mitral valve stent (1) according to claim 1 or 2 having a D-shape cross section.

4. Mitral valve stent (1) according to anyone of the previous claims wherein the sub annular anterior side height added to the engagement member length is equivalent or shorter than the sub annular posterior side height.

5. Mitral valve stent (1) according to anyone of the previous claims wherein said engagement member (4-9) is oriented in an opposite direction, i.e. equal or close to 180°, when the stent (1) is in an expanded state.

6. Mitral valve stent (1) according to anyone of the previous claims wherein said engagement member (4-9) is an integral part of the stent (1).

7. Mitral valve stent (1) according to anyone of the previous claims wherein said engagement member (4-9) and said anterior side (2) are linked to the stent body at least through a single point.

8. Mitral valve stent (1) according to anyone of the previous claims wherein said engagement member (4-9) comprises a foldable portion (5) that is made of at least one wavy line (6,6').

9. Mitral valve stent (1) according to claim 8 comprising two parallel wavy lines (6,6') wherein at least one wave of the first line (6) is linked to one wave of the second line (6').

10. Mitral valve stent (1) according to anyone of the previous claims wherein said engagement member (4-9) comprises a free end (8) that is flared.

11. Mitral valve stent (1) according to anyone of the previous claims comprising a anterior native valve locking system made of at least one self-orienting extension body (10,10'), preferably two, located within said sub annular anterior side (2), said extension body (10,10') being coincident with said sub annular anterior side (2) when the stent (1) is collapsed and which is oriented in another direction when the stent (1) is in an expanded state.

12. Mitral valve stent (1) according to claim 11 wherein said extension body (10,10') is oriented at approximately 30° towards proximal end of said sub annular anterior side (2) when the stent (1) is in an expanded state.

13. Mitral valve stent (1) according to anyone of the previous claims comprising several engagement members (4-9).

14. Mitral valve stent (1) according to claim 13 comprising one native posterior leaflet engagement member (11) that is located on the stent posterior side (3).

15. Mitral valve stent (1) according to claim 14 comprising a posterior native valve locking system made of at least one self-orienting extension body (10,10'), preferably two, located within said posterior side (3), said extension body (10,10') being coincident with said sub annular posterior side (3) when the stent (1) is collapsed and which is oriented in another direction when the stent(1) is in an expanded state.

16. Mitral valve stent (1) according to anyone of the previous claims comprising a anterior native valve locking system made of at least one self-orienting extension body (10,10'), located within said sub annular anterior side (2), said extension body (10,10') being coincident with said sub annular anterior side (2) when the stent (1) is collapsed and which is oriented in another direction when the stent (1) is in an expanded state; said stent (1) furthermore comprising a posterior native valve locking system made of at least one self-orienting extension body (10,10') located within said posterior side (3), said extension body (10,10') being preferably located at the middle height of the posterior part and being coincident with said sub annular posterior side (3) when the stent (1) is collapsed and which is oriented in another direction when the stent(1) is in an expanded state.
